(19) Europäisches Patentamt
European Patent Office
Office européen des brevets

(11) Veröffentlichungsnummer: **0 320 451**
**A2**

(12) **EUROPÄISCHE PATENTANMELDUNG**

(21) Anmeldenummer: **88810821.4**

(22) Anmeldetag: **30.11.88**

(51) Int. Cl.4: **C 07 C 37/08**
C 07 C 179/047

(30) Priorität: **09.12.87 CH 4800/87**

(43) Veröffentlichungstag der Anmeldung:
**14.06.89 Patentblatt 89/24**

(84) Benannte Vertragsstaaten:
**BE CH DE ES FR GB IT LI NL**

(71) Anmelder: **CIBA-GEIGY AG**
**Klybeckstrasse 141**
**CH-4002 Basel (CH)**

(72) Erfinder: **Clausen, Martin**
**Birchstrasse 72**
**CH-8057 Zürich (CH)**

**Rys, Paul, Prof. Dr.**
**In der Looren 51**
**CH-8053 Zürich (CH)**

**Wang, Junkuan,**
**Habsburgerstrasse 28/44**
**CH-8037 Zuerich (CH)**

(54) **Verfahren zur Herstellung von alkylsubstituierten Phenolen oder Naphtholen.**

(57) Verfahren zur Herstellung von alkylsubstituierten Phenolen oder Naphtholen, indem man die entsprechenden Dialkylbenzole bzw. -naphthaline bei höheren Temperaturen von beispielsweise 70 bis 130°C mit Sauerstoff oder Sauerstoff abgebenden Verbindungen, in Abwesenheit von Lösungsmitteln und in Gegenwart eines Alkali- oder Erdalkalimetallsalzes einer organischen Carbonsäure mit 5 bis 14 Kohlenstoffatomen zu den entsprechenden 2,6-Dialkylnaphthalin- bzw. 1,4-Dialkylphenylmonohydroperoxiden oxidiert und diese anschliessend in üblicher Weise hydrolysiert.

Das Verfahren führt zu reinen, leicht isolierbaren Produkten in guter Ausbeute. Die erfindungsgemässen Verfahrensprodukte sind wertvolle Vor-und Zwischenprodukte für unter anderem Farbstoffe, Kunststoffe oder Pharmazeutika.

EP 0 320 451 A2

**Beschreibung**

**Verfahren zur Herstellung von alkylsubstituierten Phenolen oder Naphtholen**

Die vorliegende Erfindung betrifft ein neues Verfahren zur Herstellung von alkylsubstituierten Phenolen oder Naphtholen durch Oxidation entsprechender Dialkylbenzole oder -naphthaline und anschliessende übliche Hydrolyse der als Zwischenprodukte entstehenden Monohydroperoxide.

Gegenstand der vorliegenden Erfindung ist daher ein Verfahren zur Herstellung von Verbindungen der Formel

(1) HO - Ar - R ,

worin Ar gegebenenfalls substituiertes Phenylen oder Naphthylen und R $C_1$-$C_5$-Alkyl ist, das dadurch gekennzeichnet ist, dass man Dialkylarylverbindungen der Formel

(2) $R_1$ - Ar - $R_2$ ,

worin $R_1$ und $R_2$ unabhängig voneinander $C_1$-$C_5$-Alkyl jedoch nicht gleichzeitig Methyl oder $C_5$-Alkyl sind und Ar die angegebene Bedeutung hat, in Abwesenheit von Lösungsmitteln bei Temperaturen von 70 bis 130°C mit Sauerstoff oder Sauerstoff abgebenden Verbindungen in Gegenwart eines Alkali-oder Erdalkalimetallsalzes einer organischen Carbonsäure mit 5 bis 14 Kohlenstoffatomen zu den entsprechenden Dialkylarylmonohydroperoxiden oxidiert und diese anschliessend zu den Verbindungen der Formel (1) in üblicher Weise hydrolysiert.

Weitere Gegenstände der vorliegenden Erfindung betreffen die verfahrensgemäss hergestellten Verbindungen der Formel (1) sowie deren Verwendung als Vor- und Zwischenprodukte zur Herstellung von beispielsweise Farbstoffen, Kunststoffen (Polymeren) oder Pharmazeutika.

Bei den erfindungsgemäss eingesetzten Dialkylarylverbindungen der Formel (2) handelt es sich um solche, in denen $R_1$ und $R_2$ Niederalkyl mit 1 bis 5 Kohlenstoffatomen bedeuten.

Die Alkylreste können gleich oder verschieden sein, wobei jedoch beide Reste nicht gleichzeitig Methyl oder $C_5$-Alkyl sind.

Beispiele für die Substituenten $R_1$ und $R_2$ sind Methyl, Aethyl, n-Propyl, Isopropyl, n-Butyl, Isobutyl, sek.-Butyl, tert.-Butyl und n-Pentyl, sowie die Isomeren des letzteren.

Bevorzugt enthalten die Verbindungen der Formel (2) also ein $C_1$-$C_5$-Alkyl und ein $C_2$-$C_4$-Alkyl als Substituenten $R_1$ und $R_2$, wobei solche Verbindungen besonders bevorzugt sind, in denen einer der Substituenten $R_1$ oder $R_2$ Alkyl mit 3 oder 4 Kohlenstoffatomen mit einem tertiären Proton in α-Stellung zum Arylsystem, wie beispielsweise Isopropyl oder sek.-Butyl ist.

Die Dialkylarylverbindungen der Formel (2) können weitere Substituenten enthalten, wie z.B. Halogen, vorzugsweise Chlor, ($C_1$-$C_4$)-Alkyl, ($C_1$-$C_4$)-Alkoxy oder Carboxylester (COOR′) (R′ = $C_1$- $C_4$-Alkyl).

Bevorzugte Verbindungen der Formel (2) entsprechen den Formeln

(3) 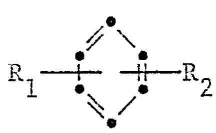 oder (4) 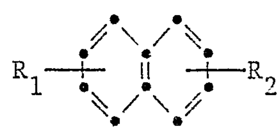 ,

worin $R_1$ und $R_2$ die angegebenen Bedeutungen haben und die Substituenten $R_1$ und $R_2$ sich in Formel (3) vorzugsweise in 1,4- und in Formel (4) vorzugsweise in 2,6-Position befinden.

Besonders geeignete Verbindungen der Formel (2) sind daher 2,6-Dialkylnaphthaline oder 1,4-Dialkylbenzole, insbesondere 2-Methyl-6-isopropylnaphthalin und 2,6-Diisopropylnaphthalin sowie 1-Methyl-4-isopropylbenzol und 1,4-Diisopropylbenzol.

Es handelt sich dabei um bekannte Verbindungen, die in bekannter Weise, z.B. durch Alkylierung der aromatischen Verbindungen hergestellt werden können.

Bei den im erfindungsgemässen Verfahren verwendeten Alkali- oder Erdalkalimetallsalzen organischer Carbonsäuren mit 5 bis 14 Kohlenstoffatomen handelt es sich vorzugsweise um Verbindungen der Formel

$$(5) \quad \left[ R_1 - \underset{\underset{R_2}{|}}{CH} - COO \right]_n M \quad ,$$

worin $R_1$ $C_1$-$C_{12}$-Alkyl und $R_2$ Wasserstoff oder $C_1$-$C_9$-Alkyl ist, die Summe der Kohlenstoffatome in den Substituenten $R_1$ und $R_2$ 3 bis 12 beträgt, M Lithium, insbesondere Natrium und Kalium, sowie Magnesium, Strontium, Barium und insbesondere Kalzium und n in Abhängigkeit von der Wertigkeit der Metalle M 1 oder 2 ist.

Als organische Carbonsäuren, deren Salze verwendet werden können, kommen beispielsweise in Betracht: n-Valeriansäure, Capronsäure, n-Heptylsäure, Caprylsäure, Pelargonsäure, Caprinsäure, Laurinsäure, Myristinsäure, 2-Ethylbuttersäure, 4-Ethylhexansäure oder 3-Ethylheptansäure und vorzugsweise 2-Ethylcapronsäure.

Die Salzbildung kann auch in-situ stattfinden, d.h. man gibt die organischen Carbonsäuren und in der Regel Alkalimetall- oder Erdalkalimetallhydroxide getrennt in das Reaktionsgemisch.

Bevorzugt verwendet werden die Kalzium- und insbesondere die Natriumsalze dieser Säuren und zwar in Mengen von 0,1 bis 1,0, vorzugsweise von 0,1 bis 0,5 Gew.-%, bezogen auf das Gewicht der Dialkylarylverbindungen der Formel (2).

Gegebenenfalls kann man für die erfindungsgemässe Oxidationsreaktion zusätzlich sog. Reaktionsinitiatoren, wie z.B. organische Radikalbildner aus der Gruppe der Peroxide, z.B. Di-tert.-butylperoxid, oder der Azo verbindungen, z.B. Azobisisobutyronitril, einsetzen. Die bei der Oxidation entstehenden Mono- oder Dihydroperoxide (2,6-Diisopropylnaphthalinmono- und -di-hydroperoxid), z.B. aus einer vorgängigen Oxidation, können ebenfalls als sog. Reaktionsinitiatoren dienen.

Die Menge dieser Initiatoren können etwa 0,1 bis 2,0, vorzugsweise 0,1 bis 1,0 Gew.-% und insbesondere 0,1 bis 0,5 Gew.-%, bezogen auf das Gewicht der Dialkylarylverbindungen, betragen.

Die Oxidation wird in Abwesenheit von Lösungsmitteln, also insbesondere von organischen Lösungsmitteln und Wasser durchgeführt. Geringe Mengen Wasser können zwar im Reaktionsgemisch vorhanden sein (wässriger Katalysator), ein wässriges Reationsmedium im Sinne einer wässrigen Lösung wird dadurch aber nicht gebildet.

Als eigentliches Oxidationsmittel kann man im erfindungsgemässen Verfahren molekularen Sauerstoff oder auch Sauerstoff enthaltende Gase, wie z.B. Luft, oder Sauerstoff abspaltende Verbindungen, wie z.B. Ozon, verwenden.

Verwendet man Sauerstoff als Oxidationsmittel, so leitet man beispielsweise 0,1 bis 1 $m^3$, vorzugsweise 0,3 bis 0,6 $m^3$ dieses Gases pro kg der Dialkylarylverbindung und pro Stunde durch das Reaktionsgemisch.

Die Reaktionszeiten liegen etwa im Bereich von 1 bis 24, bevorzugt 3 bis 8 oder 3 bis 6 Stunden. Die Reaktionstemperaturen liegen im Bereich von etwa 70 bis 130°C, bevorzugt von etwa 80 bis 120°C, und insbesondere von etwa 80 bis 110°C. Die Oxidationsreaktion wird mit Vorteil bei einem Gehalt von 20 bis 40%, beispielsweise von 35 bis 40%, an Monohydroperoxid gestoppt, da danach die Oxidationsgeschwindigkeit deutlich absinken und die Bildung von Neben- und Folgeprodukten zunehmen kann.

Nach Beendigung der Oxidation kann die Aufarbeitung des Reaktionsgemisches auf verschiedene Weise erfolgen. Die Hydrolyse der Hydroperoxide erfolgt in üblicher Weise, z.B. mit konzentrierten Mineralsäuren; die einzelnen Komponenten, wie z.B. die Zwischen- und Endprodukte oder die nichtumgesetzten Ausgangsprodukte, können dann z.B. durch Kristallisation oder Extraktion abgetrennt werden.

Für die Reaktionsgemische der Oxidation von Phenylverbindungen, z.B. der Formel (3), beginnt man die Aufarbeitung in der Regel damit, dass man diese auf etwa 50 bis 60°C abkühlt und dann mit einem niederen Alkohol, insbesondere Methanol,versetzt. In dieser alkoholischen (methanolischen) Lösung kann man dann in üblicher Weise die Hydrolyse vornehmen, indem man unter kräftigem Rühren und Temperaturkontrolle eine konzentrierte Mineralsäure, wie Schwefel- oder insbesondere Salzsäure, in katalytischen Mengen (z.B. 0,5 bis 5 Gew.-%, bezogen auf das Gewicht der Lösung) hinzugibt und die Hydrolyse solange durchführt, bis keine Hydroperoxide mehr nachweisbar sind. Das Lösungsmittel wird dann vollständig entfernt; der Rückstand wird in Petroläther (Siedebereich 30 bis 50°C) aufgenommen und die resultierende Lösung mit einer wässrigen Alkalihydroxidlösung, insbesondere einer Natrium- oder Kaliumhydroxidlösung, extrahiert. In der organischen Phase bleiben im wesentlichen die nichtumgesetzten Ausgangsprodukte zurück, die nach entsprechender Reinigung wieder in den Oxidationsprozess zurückgeführt werden können.

Die wässrige Phase kann gegebenenfalls mit Petroläther gewaschen werden; danach wird sie durch Zugabe einer Säure, insbesondere einer starken Mineralsäure, wie Schwefel- oder Salzsäure, auf einen pH-Wert von etwa 1 bis 2 eingestellt und mit Petroläther (30 bis 50°C) extrahiert.

Nach dem Trocknen der Petrolätherphase mit z.B. Kaliumhydrogencarbonat oder Natriumsulfat wird der Petroläther vollständig abdestilliert; man erhält das Alkylphenol als Rohprodukt, das aus Petroläther (30 bis 50°C) umkristallisiert werden kann (Reinheit: etwa 99,0%, Analyse mittels Hochdruckflüssigchromatographie - HPLC).

Für die Reaktionsgemische der Oxidation von Naphthalinverbindungen, z.B. der Formel (4), wird nach Beendigung der Oxidationsreaktion das Reaktionsgemisch auf etwa 50 bis 60°C abgekühlt und dann mit einem niederen Alkohol, insbesondere Methanol, versetzt. Durch diese Alkoholzugabe sinkt die Temperatur des Reaktionsgemisches weiter, dabei fällt nichtumgesetzte Dialkylarylverbindung als feinkristalliner Niederschlag aus. Durch Abkühlen auf schliesslich etwa 0°C kann der grösste Teil dieser Verbindung auskristallisiert und so abgetrennt werden. Das Filtrat (methanolische Lösung) wird danach der sauren Hydrolyse unterworfen, indem man z.B. konzentrierte Mineralsäuren, wie Schwefel- und insbesondere Salzsäure, in katalytischen Mengen (z.B. 0,5 bis 3 Gew.-%, bezogen auf das Gewicht des Filtrats) verwendet und dazu die alkholische (methanolische) Lösung kurzzeitig (10 bis 40 Minuten) unter Rühren auf Temperaturen von 40 bis 80°C, beispielsweise 60°C, erhitzt. Die Hydrolyse kann gegebenenfalls auch mit sauren Festkörperkatalysatoren, insbesondere sauren Ionenaustauschern, durchgeführt werden.

Danach wird der Alkohol (Methanol) abgetrennt und der Rückstand wird mit einem apolaren Lösungsmittel (z.B. Hexan, Heptan, Petrolether) versetzt und auf ca. 60°C erwärmt. Dann werden die ungelösten Teile

abgetrennt, mit einem inerten Lösungsmittel (Petrolether) gewaschen und schliesslich getrocknet. Bei diesem Produkt handelt es sich um die Dihydroxynaphthylverbindung, die in sehr guter Reinheit erhalten wird.

Aus dem Filtrat, das nach dem Abtrennen dieser Dihydroxyarylverbindung anfällt, kristallisiert beim Abkühlen das alkylsubstituierte Naphthol aus, das ebenfalls abgetrennt und getrocknet wird (Reinheit: etwa 98%, HPLC).

Durch Umkristallisieren aus Petrolether oder Hexan kann diese Verbindung falls notwendig weiter gereinigt werden.

Die Vorteile des erfindungsgemässen Verfahrens liegen einerseits in der Vermeidung von Schwermetallkatalysatoren, andererseits in den höheren Reaktionstemperaturen und damit kürzeren Reaktionszeiten, die man ohne die Anwesenheit von organischen Lösungsmitteln und Wasser als Reaktionsmedium erreichen kann, und schliesslich auch in der leichten Trennung der erfindungsgemäss erhaltenen alkylsubstituierten Phenole oder Naphthole von den als Ausgangsverbindung eingesetzten Dialkylarylverbindungen (so fallen z.B. die Dialkylnaphthalinverbindungen bei Aufnahme des Reaktionsgemisches in z.B. Methanol aus), sowie auch von den als Zwischenprodukte auftretenden Mono- und Dihydroperoxiden der genannten Ausgangsverbindungen.

Die verfahrensgemässen Produkte fallen in guter Ausbeute und Reinheit an. Sie sind geeignete Vor- und Zwischenprodukte für die Herstellung von beispielsweise Farbstoffen, Polymeren, synthetischen Fasern oder Pharmazeutika.

Die nachfolgenden Beispiele erläutern das erfindungsgemässe Verfahren, ohne es jedoch auf diese Beispiele zu beschränken. Teile und Prozente beziehen sich, sofern nicht anders angegeben, auf das Gewicht. Die Temperatur ist in Celsiusgraden angegeben.

Beispiel 1

10 g 2,6-Diisopropyl-naphthalin (durch gaschromatographische (GC) Analyse ermittelte Reinheit 97%) werden in einem 100ml-Vierhalskolben, versehen mit Rührer, Thermometer, Rückflusskühler und Gaseinlassfritte auf l00° C aufgeheizt. Unter intensivem Rühren gibt man 0,1 g einer Lösung (Katalysatormischung) aus 4 Teilen 2-Ethylcapronsäure, 2 Teilen Natriumhydroxid und 5 Teilen Wasser, dann 0,1 g Di-tert.-butylperoxid hinzu und startet die Sauerstoffzufuhr aus einer Gasdruckflasche in einer Menge von 80 ml/min. Nach 3 Stunden stoppt man die Sauerstoff- und Wärmezufuhr. Die Analyse des Reaktionsproduktes mittels HPLC ergibt neben unverändertem Ausgangsmaterial (2,6-Diisopropylnaphthalin) Oxidationsprodukte, die zu 91% aus den entsprechenden Hydroperoxiden (2,6-Diisopropylnaphthalin-Monohydroperoxid und 2,6-Diisopropylnaphthalin-Dihydroperoxid) bestehen. Das Gewichtsverhältnis von Monohydroperoxid zu Dihydroperoxid beträgt 9 : 1.

Zu dem auf etwa 60° C abgekühlten Reaktionsprodukt gibt man dann 40 g Methanol. Die methanolische Lösung wird tropfenweise unter Temperaturkontrolle und kräftigem Rühren mit 1 ml konzentrierter Salzsäure versetzt. Während 30 Minuten wird bei 60° C gerührt. Danach werden im Reaktionsprodukt keine Peroxide mehr nachgewiesen. Durch Kühlen der Lösung auf ca. 4° C kristallisiert der grösste Anteil des nicht umgesetzten 2,6-Diisopropyl-naphthalins und kann abgetrennt werden. Nach Trocknen unter Hochvakuum erhält man 4,7 g des 2,6-Diisopropyl-naphthalins (Reinheit: 98%, HPLC).

Von der verbleibenden Lösung wird Methanol entfernt. Den Rückstand versetzt man mit 200 g n-Hexan und das verbleibende Lösungsmittelproduktgemisch wird auf 60° C erwärmt. Ungelöste Anteile dieses Gemisches werden abgetrennt, mit Hexan und Petrolether (110 bis 140° C) gewaschen und getrocknet. Man erhält 0,21 g 2,6-Dihydroxy-naphthalin (Reinheit: 97%, HPLC).

Aus dem Filtrat, das beim Abtrennen des 2,6-Dihydroxy-naphthalins anfällt, kristallisiert während des Abkühlens auf ca. 4° C flockiges, voluminöses 6-Hydroxy-2-isopropyl-naphthalin. Nach dessen Abtrennen und Trocknen erhält man 2,35 g (Reinheit: 99%, HPLC).

Eine weitere Fraktion (0,2 g) erhält man nach Einengen des Filtrates auf dieselbe Weise. Die Ausbeute an 6-Hydroxy-2-isopropyl-naphthalin, bezogen auf das Monohydroperoxid, beträgt 90%. Der Schmelzpunkt der Verbindung beträgt: 110° C.

Analog kann man auch 2-Methyl-6-isopropyl-naphthalin zum 6-Hydroxy-2-methylnaphthalin oxidieren.

Anstelle der genannten Katalysatormischung kann man auch eine wässrige Mischung enthaltend Natrium- oder Kaliumhydroxid und eine der folgenden Carbonsäuren: n-Valeriansäure, Capronsäure, n-Heptylsäure, Caprylsäure, Pelargonsäure, Caprinsäure, Laurinsäure, Myristinsäure, 2-Ethylbuttersäure, 4-Ethylhexansäure oder 3-Ethylheptansäure, verwenden.

Beispiel 2

10 g 2,6-Diisopropyl-naphthalin werden, abgesehen von der Reaktionstemperatur, die auf 110° C eingestellt wird, wie in Beispiel 1 beschrieben oxidiert. Nach 3 Stunden werden Sauerstoff und Heizung abgestellt. Die Analyse des Reaktionsproduktes durch HPLC ergibt neben unverändertem Ausgangsmaterial (2,6-Diisopropylnaphthalin) Oxidationsprodukte, die zu 85% aus den entsprechenden 2,6-Diisopropylnaphthalinmono- bzw. -dihydroperoxiden bestehen. Das Gewichtsverhältnis von Monohydroperoxid zu Dihydroperoxid beträgt 5,4 : 1.

Die Ausarbeitung und Isolierung des gewünschten Endprodukts - 6-Hydroxy-2-isopropylnaphthalin - erfolgt durch Abtrennung von nicht umgesetztem Ausgangsmaterial und von 2,6-Dihydroxynaphthalin wie in Beispiel 1 angegeben. Die Ausbeute an 6-Hydroxy-2-isopropylnaphthalin, bezogen auf das Monohydroperoxid, beträgt: 92%.

Beispiel 3

15 g 1,4-Diisopropylbenzol (durch gaschromatographische (GC) Analyse ermittelte Reinheit 98%) werden in einem 100ml-Vierhalskolben, versehen mit Rührer, Thermometer, Rückflusskühler und Gaseinlassfritte auf 80°C aufgeheizt. Unter intensivem Rühren gibt man 0,2 g einer Lösung (Katalysatormischung) aus 4 Teilen 2-Ethylcapronsäure, 2 Teilen Natriumhydroxid und 5 Teilen Wasser, und dann 0,02 g Azobisisobutyronitril hinzu und startet die Sauerstoffzufuhr aus einer Gasdruckflasche in einer Menge von 80 ml/min. Nach 6 Stunden stoppt man die Sauerstoff- und Wärmezufuhr.

Die Analyse des Reaktionsproduktes mittels HPLC ergibt neben unverändertem Ausgangsmaterial Oxidationsprodukte, die zu 90% aus den entsprechenden Hydroperoxiden (1,4-Diisopropylbenzol-Monohydroperoxid und 1,4-Diisopropylbenzol-Dihydroperoxid) bestehen. Das Gewichtsverhältnis von Monohydroperoxid zu Dihydroperoxid beträgt 10 : 1.

Zu dem auf etwa 60°C abgekühlten Reaktionsprodukt gibt man dann 40 g Methanol. Die methanolische Lösung wird tropfenweise unter Temperatur kontrolle und unter kräftigem Rühren mit 1 ml konzentrierter Salzsäure versetzt. Während 30 Minuten wird bei 60°C gerührt. Danach werden im Reaktionsprodukt keine Peroxide mehr nachgewiesen. Aus der Lösung wird dann das Methanol entfernt. Das Reaktionsgemisch versetzt man dann mit 40 g Petrolether (Siedebereich 30 bis 50°C) und extrahiert die resultierende Lösung zweimal mit 30 g einer wässrigen Alkalilösung mit einem pH-Wert von 10 bis 11. Nachdem die vereinigten wässrigen Extrakte mit 30 g Petrolether (30 bis 50°C) gewaschen sind, wird durch Zugabe von Salzsäure auf einen pH-Wert von 1 bis 2 gestellt und mit 30 g Petrolether (30 bis 50°C) extrahiert. Die organische Phase wird über Natriumsulfat getrocknet und dann das Lösungsmittel vollständig abgezogen.

Man erhält 2,35 g 4-Isopropylphenol, was einer Ausbeute von 95%, bezogen auf das Monohydroperoxid, entspricht (Reinheit: 99% HPLC). Das Rohprodukt lässt sich aus Petrolether (30 bis 50°C) umkristallisieren. Der Schmelzpunkt beträgt: 62,7°C.

Aus der organischen Phase kann man nach der alkalischen Extraktion 1,4-Diisopropylbenzol zurückgewinnen und nach entsprechender Reinigung wieder in den Oxidationsprozess einsetzen.

In analoger Weise kann man auch 1-Methyl-4-isopropylbenzol zu p-Kresol oxidieren.

Anstelle der genannten Katalysatormischung kann man auch eine wässrige Mischung enthaltend Natrium- oder Kaliumhydroxid und eine der folgenden Carbonsäuren: n-Valeriansäure, Capronsäure, n-Heptylsäure, Caprylsäure, Pelargonsäure, Caprinsäure, Laurinsäure, Myristinsäure, 2-Ethylbuttersäure, 4-Ethylhexansäure oder 3-Ethylheptansäure, verwenden.

**Patentansprüche**

1. Verfahren zur Herstellung von Verbindungen der Formel

(1) HO - Ar - R ,

worin Ar gegebenenfalls substituiertes Phenylen oder Naphthylen und R $C_1$-$C_5$-Alkyl ist, dadurch gekennzeichnet, dass man Dialkylarylverbindungen der Formel

(2) $R_1$ - Ar - $R_2$ ,

worin $R_1$ und $R_2$ unabhängig voneinander $C_1$-$C_5$-Alkyl, jedoch nicht gleichzeitig Methyl oder $C_5$-Alkyl sind und Ar die angegebene Bedeutung hat, in Abwesenheit von Lösungsmitteln bei Temperaturen von 70 bis 130°C, mit Sauerstoff oder Sauerstoff abgebenden Verbindungen in Gegenwart eines Alkali- oder Erdalkalimetallsalzes einer organischen Carbonsäure mit 5 bis 14 Kohlenstoffatomen zu den entsprechenden Dialkylarylmonohydroperoxiden oxidiert und diese anschliessend zu den Verbindungen der Formel (1) hydrolysiert.

2. Verfahren nach Anspruch 1, dadurch gekennzeichnet, dass man die Oxidation in Abwesenheit organischer Lösungsmittel bei Temperaturen von 70 bis 130°C mit Sauerstoff oder Sauerstoff abgebenden Verbindungen in Gegenwart eines Alkali- oder Erdalkalimetallsalzes einer organischen Carbonsäure mit 5 bis 14 Kohlenstoffatomen durchführt.

3. Verfahren nach den Ansprüchen 1 oder 2, dadurch gekennzeichnet, dass die Diarylverbindungen der Formel (2), 2,6-Dialkylnaphthaline oder 1,4-Dialkylbenzole sind, deren Alkylsubstituenten $R_1$ und $R_2$ die in Anspruch 1 angegebenen Bedeutungen haben.

4. Verfahren nach Anspruch 3, dadurch gekennzeichnet, dass die Diarylverbindungen der Formel (2) als Alkylsubstituenten $R_1$ und $R_2$ $C_1$-$C_5$-Alkyl enthalten, wobei vorzugsweise einer der Substituenten $R_1$ oder $R_2$ $C_3$- oder $C_4$-Alkyl mit einem tertiären Proton in $\alpha$-Stellung zum Arylsystem ist.

5. Verfahren nach Anspruch 4, dadurch gekennzeichnet, dass die Diarylverbindungen der Formel (2) 2-Methyl-6-isopropylnaphthalin, 2,6-Diisopropylnaphthalin, 1-Methyl-4-isopropylbenzol oder 1,4-Diisopropylbenzol sind.

6. Verfahren nach mindestens einem der Ansprüche 1 bis 5, dadurch gekennzeichnet, dass die Alkali- oder Erdalkalimetallsalze der organischen Carbonsäuren der Formel

$$(5) \qquad \left[ R_1 - \underset{\substack{| \\ R_2}}{CH} - COO \right]_n M$$

entsprechen, worin $R_1$ $C_1$-$C_{12}$-Alkyl und $R_2$ Wasserstoff oder $C_1$-$C_9$-Alkyl ist, die Summe der Kohlenstoffatome in den Substituenten $R_1$ und $R_2$ 3 bis 12 beträgt, M Lithium, insbesondere Natrium oder Kalium, sowie Magnesium, Strontium oder insbesondere Kalzium und n in Abhängigkeit von der Wertigkeit der Metalle M 1 oder 2 ist.

7. Verfahren nach Anspruch 6, dadurch gekennzeichnet, dass die Menge der Erdalkali- und insbesondere der Alkali-metallsalze der organischen Carbonsäuren 0,1 bis 1,0 Gew.-%, vorzugsweise 0,1 bis 0,5 Gew.-%, bezogen auf das Gewicht der Dialkylarylverbindungen der Formel (2) beträgt.

8. Verfahren nach mindestens einem der Ansprüche 1 bis 7, dadurch gekennzeichnet, dass die Oxidation bei Temperaturen von 80 bis 120° C durchgeführt wird.

9. Die nach dem Verfahren nach mindestens einem der Ansprüche 1 bis 8 erhaltenen alkylsubstituierten Phenole oder Naphthole.

10. Verwendung der alkylsubstituierten Phenole oder Naphthole nach Anspruch 9 als Vor- oder Zwischenprodukte zur Herstellung von Farbstoffen, Polymeren, synthetischen Fasern oder Pharmazeutika.